Europäisches Patentamt

European Patent Office

Office européen des brevets

(19)

(11) Veröffentlichungsnummer: **0 191 152 B1**

(12) **EUROPÄISCHE PATENTSCHRIFT**

(45) Veröffentlichungstag der Patentschrift:
22.03.89

(51) Int. Cl.⁴: **A 61 F 2/30**

(21) Anmeldenummer: 85114682.9

(22) Anmeldetag: 19.11.85

(54) Metallisches Knochenimplantat.

(30) Priorität: 08.01.85 CH 61/85

(43) Veröffentlichungstag der Anmeldung:
20.08.86 Patentblatt 86/34

(45) Bekanntmachung des Hinweises auf die Patenterteilung:
22.03.89 Patentblatt 89/12

(84) Benannte Vertragsstaaten:
AT DE FR GB IT

(56) Entgegenhaltungen:
EP-A- 0 038 902
DE-A- 2 313 678
DE-A- 2 404 214
DE-A- 2 845 231
DE-A- 3 036 520
DE-A- 3 416 471
DE-C- 923 383
GB-A- 2 142 544
US-A- 3 605 123

REVUE TECHNIQUE SULZER, April 1974, Seiten
235-245, Winterthur, CH; M. SEMLITSCH: "L'evolution
technique des articulations coxo-fémorales
artificielles"

(73) Patentinhaber: GEBRÜDER SULZER
AKTIENGESELLSCHAFT, Zürcherstrasse 9,
CH-8401 Winterthur (CH)

(72) Erfinder: Koch, Rudolf, Oberdorfstrasse 229,
CH-8267 Berlingen (CH)
Erfinder: Frey, Otto, Wallrütistrasse 56,
CH-8400 Winterthur (CH)

(74) Vertreter: Dipl.-Ing. H. Marsch Dipl.-Ing. K. Sparing
Dipl.-Phys.Dr. W.H. Röhl Patentanwälte,
Rethelstrasse 123, D-4000 Düsseldorf 1 (DE)

## Beschreibung

Die Erfindung betrifft ein metallisches Knochenimplantat, insbesondere Kniegelenk-Endoprothese, bei dem ein Teil der Oberfläche als Gleitlagerfläche dient, während an einen anderen Teil Knochengewebe anwachsen soll, wobei das Grundmaterial des Implantates eine Co-Basis-Legierung ist, auf dessen zum Anwachsen von Knochengewebe bestimmten Oberflächenteil ein aus Titan, Tantal, Niob oder Legierungen mit einem dieser Metalle als Basiswerkstoff bestehender Strukturkörper befestigt ist.

Wegen ihrer guten technisch-mechanischen Eigenschaften – beispielsweise ihrer Festigkeit und ihrer Gleiteigenschaften – werden in der Gelenkendo-Prothetik Co-Basis-Legierungen für Knochenimplantate der vorstehend genannten Art bevorzugt. Sollen derartige Implantate ohne Zwischenschaltung eines Knochenzementbettes durch An- oder Einwachsen von Gewebe im Knochen fixiert werden, so ergeben sich Schwierigkeiten in der Haftung zwischen Knochen und Implantat und als Folge davon bei der Fixierung des Implantates im Knochen aufgrund des wenig gewebefreundlichen «Verhaltens» der genannten Co-Legierungen, an die ein Anwachsen von Gewebe nur sehr schwer stattfindet.

Aus der DE-A-30 36 520 ist es daher bekannt, zur Förderung des Anwachsens von Gewebe, metallische Substrate aus rostfreiem Stahl und Co-Legierungen sowie aus Titan, Tantal, Niob oder Legierungen dieser Metalle mit einem Strukturkörper aus Metalldrähten durch metallische Bindungen fest zu verbinden; der Strukturkörper kann aus den gleichen Metallen wie das Substrat bestehen. Die Verwendung unterschiedlicher Materialien für Substrat und Strukturkörper derselben Prothese wird dabei nicht ausdrücklich angesprochen.

In dem US-Patent 3 605 123 wird ein Knochenimplantat beschrieben, das eine poröse Oberflächenschicht aufweist; diese besteht jedoch vorzugsweise aus dem gleichen Metall wie der massive Kern. Als geeignete Metalle werden – neben Vitallium – erwähnt: Rostfreier Stahl, Titan und Tantal.

Neben dem gegenwärtigen Stand der Technik gelten Titan, Tantal, Niob und ihre Legierungen als gewebefreundlich und werden bevorzugt für Kontaktflächen zu Körpergeweben verwendet.

Aufgabe der Erfindung ist es, ein Implantat zu schaffen, bei dem einerseits die guten Gleiteigenschaften von Co-Basis-Legierungen genutzt werden, andererseits jedoch die schlechte Körperverträglichkeit dieser Werkstoffe, die zu Allergie-Reaktionen führen können, zumindest weitgehend beseitigt wird. Diese Aufgabe wird mit der Erfindung dadurch gelöst, daß zwischen dem Strukturkörper und dem als Gußteil ausgebildeten Grundmaterial des Implantates auf dem zum Anwachsen von Knochengewebe bestimmten Oberflächenteil eine auf dem Grundmaterial festhaftende, metallische Beschichtung ebenfalls aus Titan, Tantal, Niob oder Legierungen mit einem dieser Metalle als Basiswerkstoff angeordnet ist.

Eine festhaftende Beschichtung aus den genannten gewebefreundlichen Materialien läßt sich auf dem Co-Basis-Grundmaterial beispielsweise durch an sich bekannte Verfahren, wie galvanisches Aufbringen, Aufdampfen oder Flamm- bzw. Plasmaspritzen, niederschlagen; die Dicke der Beschichtung beträgt dabei vorzugsweise mindestens 0,05 mm. Der Strukturkörper kann in bekannter Weise ein Wellblech, ein Metallgitter (EP-A-0038 902 oder DE-B-24 04 214) oder ein Drahtgewebe (De-A-30 36 500) sein und gegebenenfalls aus mehreren Lagen aufgeschichtet sein.

Vorteilhaft, aber nicht zwingend notwendig, ist dabei, wenn der Strukturkörper das gleiche Material ist – oder mindestens das gleiche Basis-Material enthält – wie die Beschichtung. Weiterhin hat sich bewährt, wenn seine Haftung auf der Beschichtung über punktuelle metallurgische Kontaktstellen erfolgt, die beispielsweise durch Punktschweißen erzeugt werden. Derartige metallurgische Kontaktstellen ergeben eine gute Haftung, ohne daß das Substrat oder Grundmaterial thermischen Belastungen ausgesetzt werden muß, die sein Gefüge verändern und seine gewünschten mechanischen Eigenschaften verschlechtern.

Im folgenden wird die Erfindung anhand eines Ausführungsbeispiels im Zusammenhang mit der Zeichnung näher erläutert.

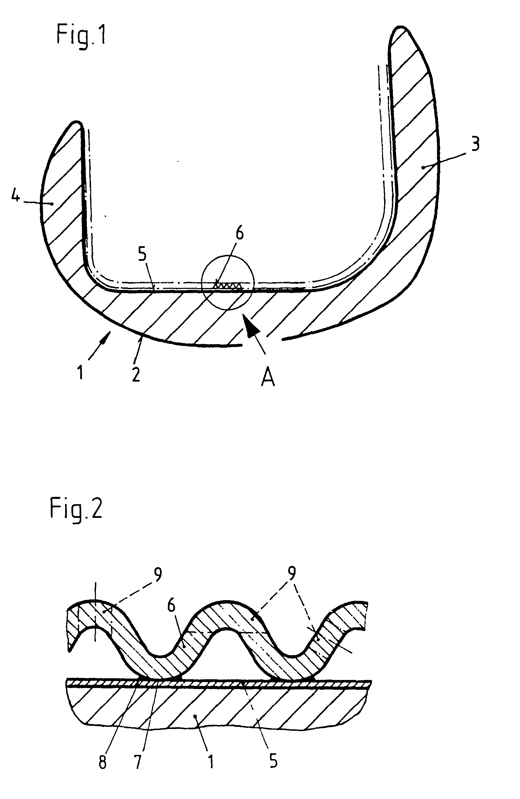

Fig. 1 zeigt in einem Schnitt parallel zur Femurachse in schematischer Darstellung eine Femurkondylenprothese für ein Kniegelenk;

Fig. 2 ist in vergrößertem Maßstab das Detail A aus Fig. 1.

Der an seiner Außenseite in Form und Abmessungen natürlichen Femurkondylen nachgebildete Prothesenteil 1 besteht aus einer Co-Basis-Gußlegierung. Das Grundmaterial, das wegen seiner Gleit- und Festigkeitseigenschaften die Grundlage des Prothesenteils 1 bildet, ist dazu bestimmt, mit seiner polierten Lauffläche 2 sich relativ zu und auf einer entsprechenden Gegenfläche eines nicht gezeigten Tibiateils zu bewegen. Die Gegenfläche besteht, wie allgemein üblich, vorzugsweise aus Kunststoff, beispielsweise aus Polyäthylen. Zwischen dem nach anterior gelegenen Schild 3, der als Gleitfläche für eine natürliche oder künstliche Patella dient, und dem hinteren Kondylenbogen 4 ist die Innenseite des Prothesenteils 1 U-förmig ausgebildet, um zementfrei in einer Art Klemmsitz auf dem nicht dargestellten Femurknochen fixiert zu werden.

Die Oberfläche des U-Bogens steht daher in direktem Kontakt mit dem Knochengewebe; um einen festen Sitz der Prothese zu gewährleisten, wird eine möglichst innige Verbindung zwischen dem Knochen und dem Prothesenteil 1 angestrebt. Dieser ist daher im Bereich seines U-Bogens zunächst mit einer Beschichtung 5 versehen, die beispielsweise aus Reintitan besteht. Die Beschichtung 5 ist z.B. nach einem der üblichen Flamm- oder Plasmaspritzverfahren hergestellt und hat eine Dicke von 0,05–1 mm.

Als Oberflächenstruktur trägt die Beschichtung 5 ein wellblechartig geformtes Blech 6, das mit der Beschichtung 5 in einer Reihe von Auflagepunkten 7 durch lokale Schweißungen 8 verbunden ist. Das Blech 6 ist ebenfalls aus Reintitan gefertigt und besitzt eine Dicke von z.B. 1 mm. In seinen Flanken und/oder in seinen Wellenkämmen ist das Blech 6 mit Durchbrüchen 9 versehen, die ein Einwachsen des Knochengewebes ermöglichen sollen. Die minimalen Abmessungen im Querschnitt der Durchbrüche betragen daher mindestens 0,1 mm.

**Patentansprüche**

1. Metallisches Knochenimplantat, insbesondere Kniegelenk-Endoprothese, bei dem ein Teil der Oberfläche als Gleitlagerfläche dient, während an einen anderen Teil Knochengewebe anwachsen soll, das Grundmaterial des Implantates eine Co-Basis-Legierung ist, auf dessen zum Anwachsen von Knochengewebe bestimmten Oberflächenteil ein aus Titan, Tantal, Niob oder Legierungen mit einem dieser Metalle als Basiswerkstoff bestehender Strukturkörper (6) befestigt ist, dadurch gekennzeichnet, daß zwischen dem Strukturkörper (6) und dem als Gußteil ausgebildeten Grundmaterial (1) des Implantates auf dem zum Anwachsen von Knochengewebe bestimmten Oberflächenteil eine auf dem Grundmaterial festhaftende, metallische Beschichtung (5) ebenfalls aus Titan, Tantal, Niob oder Legierungen mit einem dieser Metalle als Basiswerkstoff angeordnet ist.

2. Knochenimplantat nach Anspruch 1, dadurch gekennzeichnet, daß die Schichtdicke der Beschichtung (5) mindestens 0,05 mm beträgt.

3. Knochenimplantat nach Anspruch 1 oder 2, dadurch gekennzeichnet, daß der Strukturkörper (6) über punktuelle metallurgische Kontaktstellen (8) auf der Beschichtung (5) haftet.

**Claims**

1. A metal bone implant, more particularly a knee joint replacement, in which some of the surface is a plain bearing surface while bone tissue is intended to grow on another part of the implant, the parent material of the implant being a Co-based alloy, there being secured to the surface part intended for the ongrowth of bone tissue a structured member (6) of titanium, tantalum, niobium or alloys having one of these metals as base substance, characterised in that between the structured member (6) and the parent material (1), in the form of a casting, of the implant a metal coating (5) which adheres to the parent material and which also consists of titanium, tantalum, niobium or alloys having one of these materials as base substance is disposed on the surface part intended for the ongrowth of bone tissue.

2. A bone implant according to claim 1, characterised in that the layer thickness of the coating is at least 0,05 mm.

3. A bone implant according to claim 1 or 2, characterised in that the structured member (6) sticks to the coating (5) by way of spot metallurgical contact zones.

**Revendications**

1. Implant osseux métallique, notamment endoprothèse rotulienne, dans lequel une partie de la surface sert de surface de portée coulissante, tandis que du tissu osseux est destiné à croître périphériquement sur une autre partie, le matériau de base de l'implant étant un alliage à base de Co, et un corps structurel (6), consistant en du titane, du tantale, du niobium ou en des alliages englobant l'un de ces métaux en tant que matériau de base, étant fixé sur la partie superficielle dudit matériau de base qui est destinée à la croissance périphérique de tissu osseux, caractérisé par le fait qu'un revêtement métallique (5) consistant également en du titane, du tantale, du niobium ou en des alliages renfermant l'un de ces métaux en tant que matériau de base, adhérant fermement au matériau de base, est intercalé entre le corps structurel (6) et le matériau de base (1) de l'implant, réalisé sous la forme d'une pièce coulée, sur la partie superficielle destinée à la croissance périphérique de tissu osseux.

2. Implant osseux selon la revendication 1, caractérisé par le fait que l'épaisseur de couche du revêtement (5) mesure au moins 0,05 mm.

3. Implant osseux selon la revendication 1 ou 2, caractérisé par le fait que le corps structurel (6) adhère au revêtement (5) par l'intermédiaire de zones de contact (8) métallurgiques ponctuelles.

Fig.1

Fig.2